(19) Europäisches Patentamt
European Patent Office
Office européen des brevets

(11) EP 1 568 736 A2

(12) EUROPEAN PATENT APPLICATION

(43) Date of publication:
**31.08.2005 Bulletin 2005/35**

(21) Application number: **04425119.7**

(22) Date of filing: **26.02.2004**

(51) Int Cl.[7]: **C08L 33/26**, C08L 29/04, C08F 261/04, C08J 3/075, A61L 27/26

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Polymekon S.r.l.**
**20144 Milano (IT)**

(72) Inventors:
- **Protopapa, Carmelo**
  **72100 Brindisi (IT)**
- **Balestrieri Gerardo**
  **20124 Milano (IT)**

(74) Representative: **Tansini, Elio Fabrizio**
**c/o Bugnion S.p.A.**
**Viale Lancetti, 19**
**20158 Milano (IT)**

(54) **A process for preparing alcoholic polyacrylamide interpolymers in the form of biocompatible hydrogels**

(57) The present invention relates to an innovative method for preparing and producing a hydrogel having properties making it suitable for use as a filler or substitute for human and animal tissue. More specifically, the invention relates to a composite material comprising a gelled interpolymer consisting of a copolymer such as a polyacrylamideimide and a polyvinyl alcohol polymer.

EP 1 568 736 A2

## Description

The present invention relates to an innovative method for preparing and producing a hydrogel having properties making it suitable for use as a filler or substitute for human and animal tissue.

This invention constitutes a development of a prior patent filed by the authors at number PCT/IB01/02721 dated 24.12.2001.

The authors have created a new polymer based on the formulations constituting the polymer disclosed in the prior patent.

The innovative properties of the polymer forming the subject matter of this invention are specified below.

The term "*gel*" is derived from gelatin, meaning a protein prepared by hydrolysis of collagen. Gels include a very wide range of substances all having in common the ability to absorb large quantities of liquid while maintaining their elastic properties.

To date, there is no complete and accurate method of classifying a material as a gel.

A gel consists of two separate, interpenetrated phases: a liquid phase and a solid phase usually consisting of a polymer network. If the liquid phase is an aqueous solution, the gel is referred to as a *hydrogel.* In this case, the polymer chain may include ionizable groups (electrolyte polymers) with their own dissociation constant, whilst the interstitial solution includes small ions (counterions), produced by the dissociation, which contribute to keeping the electrical neutrality of the gel.

Numerous parts of the human body consist of gel-like aggregates. These include, for example, vitreous humour and the cornea of eyes, synovial fluid, etc. Other tissues, such as cartilage, the dermis, tendons and so on, although they have a cell component, may be broadly classified as two-phase systems where an interstitial fluid permeates a solid matrix. In the case of the dermis, the solid matrix is made up of fibrous proteins which are in turn immersed in a highly charged, amorphous polysaccharide base.

The rheological and functional properties of these tissues, sometimes very complex, may be explained by their composition.

Many polymers are currently used in medical devices. The fields of major application are flow control of blood and other body fluids (catheters, cannulas, drainage devices), joint surfaces in orthopaedic prostheses, contact and intraocular lenses, membranes for administering drugs, coatings for implantable electronic devices and sensors, tissue regeneration, cavity filling, heart valves, vascular prostheses, bioartificial organs (medical devices combining synthetic materials and living tissue or cells). Examples are the following: polyglygolic acid (PGA) for biodegradable sutures and medullary plates, polylactic acid (PLA) for artificial ligaments and controlled release of drugs; poly-hydroxy ethyl methacrylate (PHEMA) for contact lenses, etc.

The main advantages of gelled polymers over other classes of materials are: better biocompatibility; possibility of changing their physical and mechanical properties; low friction coefficients; easy processability and workability even in complex forms and structures; possibility of changing their surfaces chemically and/or physically; possibility of immobilising cells or biomolecules internally or on the surface.

The main disadvantages are: presence of substances that can be released into body tissue (monomers, catalysts, additives, etc.); the ease with which they absorb water and biomolecules from the surrounding environment (even in applications where this is not required); poor mechanical properties; and, in some cases, difficulty of sterilisation. It is well known that the final properties of a polymer depend on both its intrinsic molecular structure and on the chemical and physical properties it is subjected to. These may be engineered to a large extent by controlling the working conditions and the polymerisation reaction. The polymers used for this purpose may be natural (for example, collagen and cellulose), artificial (chemically modified) or synthetic (obtained by chemical synthesis).

The structure, composition and surface properties of the implanted polymer that is in direct contact with body tissue fluids determine the biological response of the host organism because they are responsible for stress transmission, adhesion, friction, abrasion, gas and liquid permeability, and compatibility with the surrounding corrosive organic environment. In the optimum solution, the material and the tissue should interact as appropriately as possible to maximise the effective incorporation of the material into the surrounding tissue and, hence, to guarantee stability. Diverse methods have therefore been devised to modify the surface of the polymers used in medical devices in order to optimise their specific interactions with the tissue of the host organism, that is to say, their biocompatibility, but without modifying the mechanical and functional properties of the device. Implanted materials may be subject to both passive and active degradation processes. When a polymer material is degraded, its chemical bonds are broken down, not only in the main chain but also in side groups, and the arrangement of its molecules changes. Usually, one of the main negative effects of degradation is reduced molecular weight, leading to a reduction in the mechanical properties of the material. The main causes of degradation or deterioration of polymers for biomedical use are the following: effects of sterilisation processes, effects of the biological environment and chemical effects.

Sterilisation, which destroys micro-organisms, is essential to prevent infections following implantation of a medical device. Some sterilisation methods may cause polymer degradation. When sterilising with dry heat, the temperature must vary between 160 and 190°C, which is higher than the softening point of many linear chain polymers such as, for example, polyethylene or polymethylmethacrylate.

Steam sterilisation (autoclaving) is carried out using steam at high pressure and at temperatures of between 121 and 135°C for a minimum of 17 min. This method is therefore unsuitable for polymers that may be attacked by the steam. Another sterilisation method involves the use of chemical agents in the form of a gas (ethylene or propylene oxide) or solutions at low temperature.

The biological environment inside the human body is extremely aggressive against polymers: so much so that all polymers start deteriorating to some extent soon after being implanted. The most probable cause of deterioration is ionic attack (especially attack by OH- ions) and dissolved oxygen. Usually, hydrophilic polymers deteriorate more easily than hydrophobic polymers. In the case of polymers of biological origin, such as collagen, for example, enzymatic degradation occurs more easily because the body possesses specific enzymes for that purpose (collagenase).

Body tissues include numerous molecules and cells capable of catalysing chemical reactions or of rapidly isolating, attacking and destroying foreign objects. Most polymers used in medical devices allow water to spread in the molecular structure, giving rise to hydrolytic processes. The choice of a polymer that is hydrolisable/non-hydrolisable, or non-resistant to water absorption is closely linked to the type of application required. Thus, for example, sutures and devices for the controlled release of drugs require materials that are easily degradable.

In vivo polymer degradation processes (biodegradation) are not simply the result of water absorption. Many other factors that greatly increase their speed in vivo must be taken into account. Indeed, many cells, including those involved in inflammatory processes, produce enzymes that catalyse certain degradation reactions, leading to alterations in the molecular structure of the implanted materials. Furthermore, some specific cells (phagocytes) migrate towards the zones involved in irritative and inflammatory processes caused by implanted objects, and are adsorbed to their surface which is recognised as foreign by certain proteins (antibodies for example). This mechanism leads to a rapid increase in the body's metabolic defence activities and is responsible for activating the polymer degradation processes. The degradation processes of the biomaterials vary according to the position of the implant within the host organism, the type of tissue the biomaterials are in contact with and on which the response of the immune system depends, the physical and chemical properties of the material and the general conditions of the host organism, (age, health, drugs taken, etc.).

The reactions of the human body in response to the implantation of a medical device produce effects not only in the interface zone but also in zones further away from the implanted device. In the immediate vicinity of the implant, the main effect is the absorption of proteins from the blood by the surface of the device. The host organism also responds locally in the area surrounding the implanted device. This response is divided into two stages: initially, there is inflammation because the first reaction of the organism's defence mechanism to a foreign object is a change in the microvascular structure and, hence, in the nature of the tissues. This is a followed by a reparative response in which the tissues activate certain processes in an attempt to repair the structural damage and, where possible, the functional damage.

As a general rule, if the implanted material is toxic, necrosis of the surrounding tissue ensues; if it is non-toxic and biologically inert, a fibrous capsule is formed (this response is quite rare because usually the biomaterial is not totally inert); lastly, if the material is bioactive, it stimulates a precise biological response and is gradually incorporated into the surrounding tissue. In most cases, the polymer undergoes some form of degradation and the by-products of this process are released into the tissues, considerably affecting the organism's defence mechanisms and cellular activity. If these by-products are not biologically active or toxic, they are eliminated by normal metabolic processes. If their concentration reaches high values, however, they may accumulate locally or in target organs (spleen, kidneys, lymph nodes) and give rise to acute or chronic diseases. If the by-products are toxic, on the other hand, persistent inflammation ensues, with interruption of reparative processes and, in some cases, tissue necrosis in some areas. Furthermore, the by-products of degradation processes may be confined to the release area, producing only local effects, or they may spread in the vascular system, thus producing effects on organs or tissues that are far away from the release area.

To conclude, therefore, the desirable properties of any polymer used for medical applications should be such as to enable the polymer to resist the above mentioned degradation processes to the greatest possible extent and to produce a response that is as harmless as possible, thus guaranteeing safety and effectiveness.

In the light of the foregoing, the authors have further improved the polymer disclosed by the patent PCT/IB01/02721 dated 24.12.2001 and have created a novel material forming the subject matter of the present invention.

Acryl or acrylamide hydrogels used up to now are in fact obtained from the interaction of different monomers and are therefore copolymers (see GB No.2114578; RU No.2127129 and related patents).

It is known that these hydrogels are the result of a process of copolymerisation between two compounds that are both capable of being polymerised individually. Interpolymerisation, on the other hand, occurs when a third compound is used which cannot itself be polymerised but reacts only thanks to the presence of the other two. Some resins used in paints and varnishes such as polyvinyl chloracetate with the addition of alcohols or of maleic anhydride, and

compounds including glass fibres (Fiberglas ™) are some examples of interpolymers.

This type of polymerisation differs completely from heteropolymerisation and constitutes an absolute novelty in the field of hydrogels for medical use.

The present invention has for an object to provide a gelled interpolymer with a high water content (hydrogel). The interpolymer according to the invention involves a reaction between two compounds which tend to copolymerise in themselves (see patent PCT/IB01/02721 dated 24.12.2001) and a third compound which is by itself unable to polymerise but is used to create a polymer complex with larger molecular size and better viscoelastic properties. In actual fact, this invention provides a *composite material* consisting of two different polymers (pAA-I and pVA) in order to obtain a material that possesses the properties of both its components. More specifically, it is a compound (polyacrylamide-imide) reinforced by another component (cross-linking comonomer) which traps and binds another polymer (polyvinyl alcohol) in such a way as to obtain greater cohesion and elasticity. All of this means greater resistance.

The concepts expressed in the prior patent (PCT/IB01/02721 dated 24.12.2001) have been further developed with regard to the influence of the Van der Waals forces which are active at short distance and derive from the polarisation induced by molecular electron "clouds".

It should be remembered that the average electron distribution of a molecule is uniform, whereas its instantaneous distribution is not uniform. One side of the molecule might - by chance - have a slightly higher electron charge than the other side of it, thus creating an instantaneous dipolar movement. In such a case, the instantaneous dipole would cause an adjacent molecule to adopt a dipole of different - or rather - opposite orientation, thus inducing attraction between the two molecules. These instantaneous dipoles change constantly and the higher the molecular weight is, the greater the cumulative effect of the attractive forces. This "play" on forces affects the homogeneousness of the polymer being formed and, at the same time, the uniformly distributed temperature of the product augments the movement of the molecules in the solute which, in our case, consists of monomers.

For this reason, it was essential to have an absolutely uniform dispersion of the monomers. To obtain this, optimum blending of the monomers was essential and had to be performed with the aid of a protective colloid with the highest possible "golden number", such as polyvinyl alcohol, without interfering with the three-dimensional amide-imide formation oaccording to the patent we were referring to, but, on the contrary, improving its properties of:

- being non-genotoxic,
- being non-toxic,
- not leading to sensitisation
- being highly biocompatible
- being non-allergenic
- being permanent, and
- being able to be easily removed.

On these bases, in order to improve on the product forming the subject-matter of the prior patent, we formulated and produced a new product that can be defined as an *interpolymer of PVA (polyvinyl alcohol) and PAA-I (polyacrylamide-imide) copolymer,* forming the subject-matter of this invention.

The polyvinyl alcohol used for the purposes of this invention must have an extremely high degree of purity, that is to say, it must be free of salts, aldehydes and other substances that might spoil the reactions referred to below. In fact, it not only homogenises the monomers perfectly but also takes part in the process of forming the three-dimensional cross link of the hydrogel.

The polymer chain of the polyvinyl alcohol, especially if of high molecular weight, contains small quantities of diols (which increase in direct proportion to the molecular weight), as is very well known from scientific literature (A.D. McLaren and R.J. Davis, J. Amer. Chem. Soc.68, 1134 -1946; P.J. Flory and F.S. Leutner, J. Polymer. Sci.,5,267 -1950).

The diols are formed by hydroxylation during the process of polymerisation from which the polyvinyl alcohol is derived and which consists in forming or rather adding an - OH group to each carbon atom having a double alkene bond.

The 1,2 diol or glycol group is as follows:

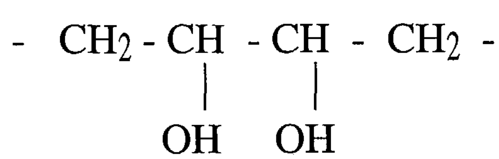

The content of the 1,2 diol glycol group was estimated at 1-2 moles per 100 moles. There are also smaller quantities of 1,3 glycol groups.

With regard to the composition of the polyvinyl alcohol, it should be remembered that the use of peroxides during polymerisation of the acetate monomer from which it derives, forms carbonyl groups, that is to say, carbonyls of the following type:

$$R=C=O$$

as demonstrated by the study of absorption spectra (see J.T. Clarke and E.R. Blout, J. Polym. Sci.,1,419-1946).

The presence of these carbonyl groups is revealed by ultraviolet spectroscopy, which, as is known, is used especially to provide this type of information. Its spectrum ranges from 200 to 400 nanometres.

The absorption of the above mentioned carbonyl groups appears at 225, 280 and 330 nanometres.

These factors must be considered in relation to the interaction of polyvinyl alcohol with the catalysts and, above all, with the oxidation treatments in an acid environment.

In this regard, studies were carried out into the oxidation of PVA with oxidising agents such as oxygen, ozone, potassium bichromate, etc., which cause degradation of the polymer.

When the polyvinyl alcohol is treated in this way, the molecular chain breaks and, at the break points, the carbonyl groups mentioned above are formed (as impurities) together with carboxyl groups (I. Sakurada and S. Matsuzava, Kobunshi Kagaku, 21, 716- 1962).

At this point it is very important to bring together all the observations made up to now in connection with the process for preparing the hydrogel we are referring to, disclosed in patent PCT/IB01/02721 of 24.12.2001, and to draw conclusions defining the work carried out. That is to say:

1. Oxidation

Looking in more detail at the process described in the patent of 24.12.2001, it will be noticed that one of the aspects that distinguishes it is the prescribed treatment with oxygen during the first step of polymerising the monomers. This treatment makes it possible to significantly slow down the polymerisation process, which is decisive to obtaining molecular chains of considerable length, or at least long enough to confer on the end product the properties required to meet our needs.

This treatment, however, also has another purpose: namely, that of oxidising all or part, depending on circumstances, of the polyvinyl alcohol chains, thus creating the carboxyl groups.

This action, however, is completed by another action, due to the presence of ammonium persulphate. This substance, as is known, breaks down into water as follows:

$$(NH_4)_2 S_2O_8 + 2H_2O \rightarrow (NH_4)_2SO_4 + H_2SO_4 + H_2O_2$$

2. Creation of an interpolymer formation

Our hydrogel is prepared with the "redox" method which, as is known, consists of a half-reaction of reduction in which a chemical species acquires electrons and its oxidation number decreases, and a half-reaction of oxidation in which electrons are yielded.

We have explained in detail how this type of product is formed by creating polymer chains "linked" to each other through activation of the double bonds of bi-reactive products. These chains are arranged in a three-dimensional formation.

In this method, the polyvinyl alcohol represents the action, in the aforementioned formation, of an interpolymer linked to the chain of one of the carbonyl monomers being formed, according to the following reaction:

chain being formed

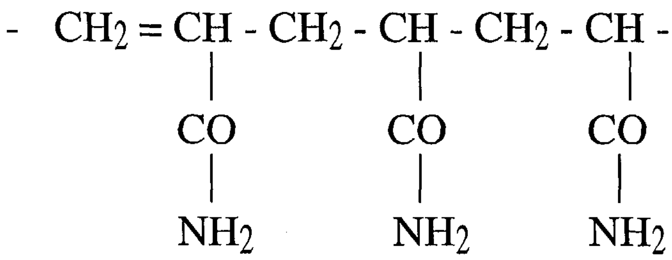

linked polyvinyl alcohol chain

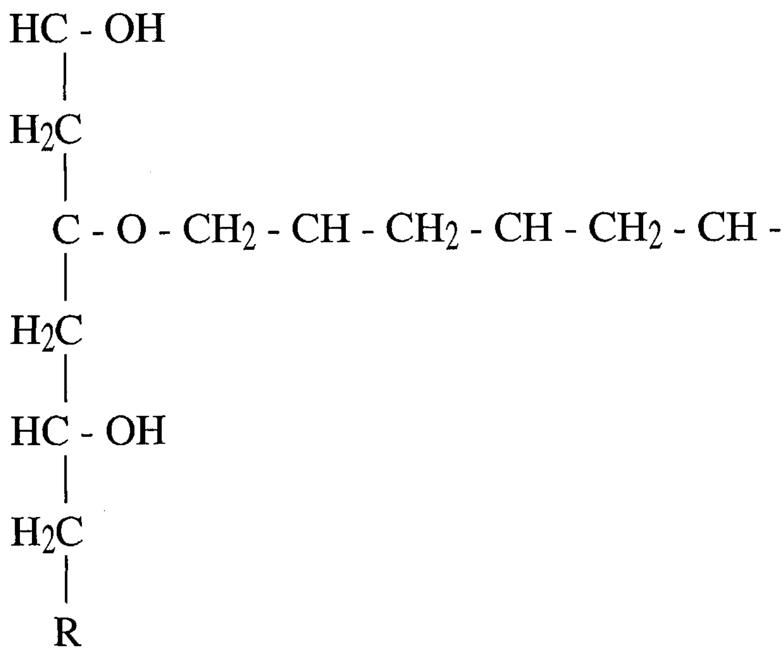

Further, in the light of the properties of polyvinyl alcohol, described above, the highly acid environment in which the reactions take place, and the calculated temperature modulation, a second reaction of the same chain is also hypothesised: that is, a reaction between the carbonyl and carboxyl groups in the polyvinyl alcohol.

Whatever the case, the new hydrogel includes a polyalcohol chain that produces an interpolymerisation process.

In view of the properties of the polyvinyl alcohol, as described above, and the pH in which the reactions take place, we cannot exclude yet another reaction between the carbonyl groups

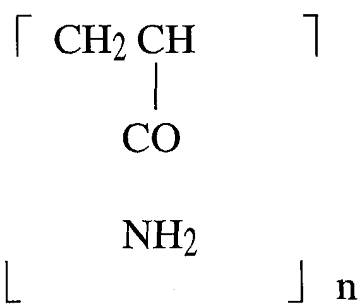

and the carboxyl groups in the polyalcohol chain

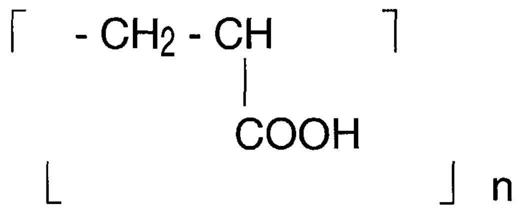

forming yet another bond

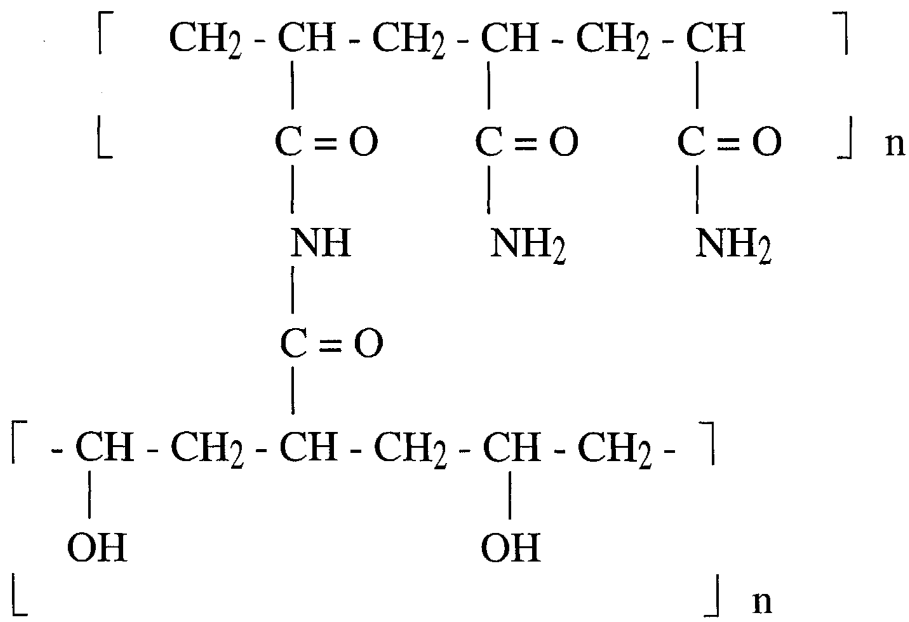

This type of cross linking and the special process for making the interpolymer according to this invention permit a more linear distribution of the polymer chains. If we assume that the polymer will be introduced subcutaneously in order to correct a tissue volume deficit, it is important that the force of application (for example, pressure on the outside of the skin) is as parallel as possible (and not perpendicular) to the chains that form the polymer, so as to increase its mechanical strength and elasticity.

In order to confirm the improvements achieved by this invention, we compared the biological parameters (biomimesis) and the chemical and physical parameters with those of the polymer disclosed by the invention PCT/IB01/02721 of 24.12.2001.

The histological test (T.F.) showed that the biocompatibility (or biomimesis) of the interpolymer is significantly improved compared to the polymer of the prior patent. The degree of resistance to degradation processes is also improved.

As regards physical properties, the rheological tests revealed a marked improvement not only in the degree of structuring (indicating a virtual parameter of the degree of defined cross linking Z) previously, Z=6-7 and currently Z=12-13, but also in molecular strength (indicating a virtual parameter of the defined bonding strength A) previously A=50-60 and currently A=97.

As regards chemical properties, the following were tested:

| | | |
|---|---|---|
| 1. | pH | = 5,5 +/- 0,5 |
| 2. | oxidizability (Kubel method) | = 1,2 +/- 0,5 |

(continued)

| | | |
|---|---|---|
| 3. | monomeric ppm value | = > 0,6 |

The gel is colourless and odourless like the polymer described in patent PCT/IB01/02721.

EXAMPLE OF INTERPOLYMERISATION

Solution consisting of:

- ✓ 20 g acrylamide
- ✓ 1 g M-bis-acrylamide
- ✓ 0.5 g E-bis-acrylamide
- ✓ 6 g PVA
- ✓ 0.04 ml ammonium persulphate
- ✓ 0.5 ml hydrogen peroxide
- ✓ 400 ml apyrogenic water

The acrylamide monomer is placed in solution with the catalyst and the complex is agitated for 50 minutes at a temperature of 50°C. This provides an acrylamide homopolymer to which cross linking agents are added while the complex is agitated and molecular $O_2$ bubbled through it for 15 minutes. After mixing the solution, PVA and another catalyst are added in order to promote the cross linking reactions while being agitated for 30 minutes.
The complex is then allowed to stand for 24 hours at 36°C.
Document PCT/IB01/02721 is incorporated by reference into the present patent application, particularly as regards a process for preparing a cross-linked acrylic polymer from water soluble acrylamide monomers. The process comprises the following steps:

- preparing an aqueous polymerising solution comprising the acrylamide monomer and catalysing agents;
- polymerising the monomers present in the polymerising solution by agitating and heating the polymerising solution. The process is characterised in that the polymerising step is performed in the presence of gaseous oxygen to obtain a cross-linked acrylic polymer.

According to the process, the polymerising solution is preferably saturated with gaseous oxygen.
According to the process, the oxygen is preferably bubbled through the polymerising solution for a length of time varying from 1 to 24 hours at a temperature between 30° and 60°C.
According to the process, the polymerising step is preferably followed by a further step of washing the polymer in aqueous medium at a temperature of between 80° and 100°C for a length of time that may vary from 3 to 5 hours.
According to the process, the washing step is preferably followed by a step of holding the polymer at a temperature of between 110° and 130°C for a length of time that may vary from 1 to 6 hours.
According to the process, the polymerisation of the monomers is preferably performed at a temperature between 30° and 80°C.
According to the process, the polymerisation of the monomers is performed for a length of time varying from 1 to 24 hours.
According to the process, the aqueous polymerising solution preferably comprises the monomers acrylamide and one or both of N,N'-methylene-bis-acrylamide and N,N'-ethylene-bis-acrylamide.
According to the process, the polymerising solution preferably also comprises ethylene-bis(oxyethylene nitrilo)-tetracetic acid.
According to the process, the polymerising step is preferably performed in the presence of metal salts having a metal cation that may be of aluminium, zirconium or titanium.
The cross-linked acrylic polymer prepared using the process described in document PCT/IB01/02721 is incorporated by reference into the present patent application.
The acrylic polymer is preferably used as a filler in aesthetic and reconstructive plastic surgery.

## Claims

1. A composite material comprising a gelled interpolymer consisting of a copolymer such as a polyacrylamide-imide and a polyvinyl alcohol polymer.

**2.** The material according to claim 1, wherein the polyacrylamide-imide can be made according to a process comprising the following steps:

- preparing an aqueous polymerising solution comprising the acrylamide monomer and catalysing agents;
- polymerising the monomers present in the polymerising solution by agitating and heating the polymerising solution in the presence of gaseous oxygen to obtain a cross-linked acrylic polymer.

**3.** The material according to claim 1, wherein the polyvinyl alcohol has a degree of hydrolysis of at least 98%, preferably 99%.

**4.** The material according to at least one of the foregoing claims from 1 to 3, wherein the catalysts may be ammonium persulphate or hydrogen peroxide.

**5.** The material according to at least one of the foregoing claims from 1 to 4, wherein the PVA is present in a quantity of between 0.001 and 10.

**6.** Use of the material according to at least one of the foregoing claims from 1 to 5 for preparing a filler for use in medicine, plastic and reconstructive surgery, dermatology and aesthetic surgery.

**7.** Use of the material according to claim 6, for preparing a material to be used as a filler or substitute for tissue or organs.